# EUROPEAN PATENT APPLICATION

(11) **EP 2 351 749 A1**
(43) Date of publication of application: **03.08.2011**
(21) Application number: 09824819.8
(22) Date of filing: 05.11.2009
(51) Int. Cl.: C07D 401/12, A61K 31/496, A61P 7/12, A61P 13/00, A61P 13/08, A61P 13/10, A61P 25/20, C07D 405/12, C07D 409/12, C07D 417/12

(54) **CARBAMATE COMPOUND OR SALT THEREOF**

(30) Priority: 06.11.2008 JP 2008285466
(71) Applicant: Astellas Pharma Inc., Tokyo 103-8411 (JP)
(72) Inventor: ISHII, Takahiro, Tokyo 103-8411 (JP); SUGANE, Takashi, Tokyo 103-8411 (JP); MUNAKATA, Ryosuke, Tokyo 103-8411 (JP); AOKI, Satoshi, Tokyo 103-8411 (JP); HIGAKI, Masahide, Tokyo 103-8411 (JP); SOMEYA, Akiyoshi, Tokyo 103-8411 (JP)
(74) Representative: HOFFMANN EITLE
(86) International application number: PCT/JP2009/068902
(87) International publication number: WO 2010/053120

(57) **Abstract**

[Problems] A compound useful as an active ingredient for a pharmaceutical composition for treating FAAH-related diseases is provided.

[Means for Solution] The present inventors have made extensive studies on compounds having an FAAH inhibitory activity, and as a result, have found that a piperazine-1-carboxylate compound, in which benzimidazol-2-ylcarbonyl, benzofuran-2-ylcarbonyl or the like binds to the 4-position of the piperazine, has an excellent FAAH inhibitory activity and further has an action to increase the effective bladder capacity, an action to ameliorate sleep disorders, an anti-diuretic action, and an analgesic activity on lower urinary tract pain including bladder pain and the like, thereby completed the present invention. The carbamate compound of the present invention has an excellent FAAH inhibitory activity and can be used as an agent for preventing and/or treating FAAH-related diseases, particularly nocturia, interstitial cystitis, painful bladder syndrome, or chronic non-bacterial prostatitis/chronic pelvic pain syndrome.

## Description

### Technical Field

The present invention relates to a carbamate compound or a salt thereof useful as an active ingredient of a pharmaceutical composition, particularly a pharmaceutical composition for treating FAAH-related diseases.

### Background Art

Urinary frequency can be roughly divided into increased daytime frequency and nocturia (Journal of Neurogenie Bladder Society, Vol. 14, 2003). Increased daytime frequency refers to a complaint in which a patient has an excessive number of incidences of urination during the day and increased daytime frequency may be conveniently defined by the number of such incidences (for example, 8 times or more) in some cases. In contrast, nocturia refers to "the complaint that the individual has to wake at night one or more times to void" and has been defined as a new kind of diseases (International Continence Society: ICS, 2002). It is thought that a variety of factors contribute to the occurrence of nocturia, including, for example, nocturnal polyuria, decreased nocturnal bladder capacity (decreased arousal threshold to urinate), sleep disorders, and the like as major factors. Herein, nocturnal polyuria refers to an increased amount of urine generated at night and is defined as a case where the nocturnal urine amount exceeds 20 (young) to 33 (elderly)% with respect to a daily urine quantity (BJU International, Vol. 90, pp. 18-20, 2002). In addition, nocturia and sleep disorders have a both-way relationship with each other, and as a result of nocturia, there is a schema which causes secondary sleep disorders while, as a result of sleep disorders, there exists a schema which leads to secondary nocturia (VOIDING DISORDERS DIGEST, Vol. 13, 2005). In any case, sleep is disturbed, reducing QOL, and this is a disease which also causes bone fractures when waking up at night for urination and then falling in the case of the elderly people.
Increased daytime frequency and nocturia are not necessarily combined, and among patients with nocturia, there are many people who are normal during the day. This is due to different causes of increased daytime frequency and nocturia. However, there are diverse causes of nocturia, such as nocturnal polyuria, decreased nocturnal bladder capacity, sleep disorders, and the like, and based on this, an anticholinergic drug which is the first-line agent for treatment of urinary frequency shows an effect in patients with increased daytime frequency, but shows an insufficient effect in patients with nocturia (Int Urogynecol J, Vol. 18, pp. 737-741, 2007).
From the above, the expectation for a drug for nocturia having a novel mechanism of action is high.

Fatty acid amide hydrolase (FAAH) is known to hydrolyze endocannabinoid to inactivate it (Prostaglandins Leukotrienes and Essential Fatty Acids, Vol. 66, pp. 143-160, 2002; British Journal of Pharmacology, Vo. 141, pp. 253-262, 2004; Nature, Vol. 384, pp. 83-87, 1996; Biochemical Pharmacology, Vol. 62, pp. 517-526, 2001). Endocannabinoid is a generic term for a biological substance that acts on a cannabinoid receptor to exhibit its physiological activity. Typical endocannabinoids are anandamide, palmitoylethanolamide, oleamide, and 2-arachidonoylglycerol and they are known to be hydrolyzed by FAAH to lose their activity. In addition, Δ9-tetrahydrocannabinol that is considered as an active ingredient of cannabis (marijuana) is known to activate a cannabinoid receptor (Current Medicinal Chemistry, Vol. 6, pp. 635-664, 1999).

In mammals, two types of cannabinoid receptor CB 1 and CB2 have hitherto been known. CB 1 is expressed in central and peripheral nervous systems, and when activated, it causes its mental action, analgesic action and the like. CB2 is expressed in immune systems, and when activated, it causes an anti-inflammatory action, an analgesic (inflammatory) action, and the like.

On the other hand, in a cystitic rat model, a cannabinoid receptor agonist increases the bladder capacity and the urination threshold (The Journal of Neuroscience, Vol. 22, pp. 7147-7153, 2002; Pain, Vol. 76, pp. 189-199, 1998): it is known that anandamide, which is one of the substrates for FAAH, is a substance causing sleep (Brain Research, Vol. 812, pp. 270-274, 1998): and side effects such as hallucination, delusion, tachycardia, orthostatic hypotension, and the like observed in the administration of a cannabinoid receptor agonist to animals are not observed when an FAAH inhibitor is administered thereto (Nature Medicine, Vol. 9, pp. 76-81, 2003). Therefore, the FAAH inhibitor is expected to be a novel agent for treating nocturia that has less risk of causing the side effects and compatibility problems.

The following compounds are known as compounds having an FAAH inhibitory activity.
Patent Document 1 discloses a compound represented by the following formula (A), as a compound having an FAAH inhibitory activity and useful for treatment of neuropathic pain or the like. 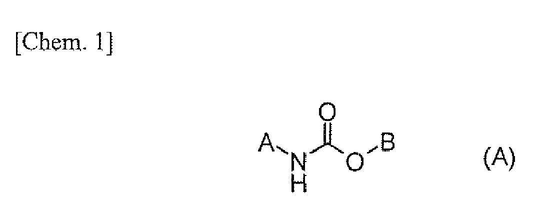 (In the formula, B means various ring groups which may be substituted, or the like, and A means phenyl which may be substituted, phenylalkyl which may be substituted, dibenzofuranyl, dibenzothienyl, naphthyl, indolyl, fluorenyl, or carbazolyl. For details, refer to the publication.)
Furthermore, Patent Document 2 discloses a compound represented by the following formula (B), as a compound having an FAAH inhibitory activity and useful for treatment of anxiety, pain, or the like. 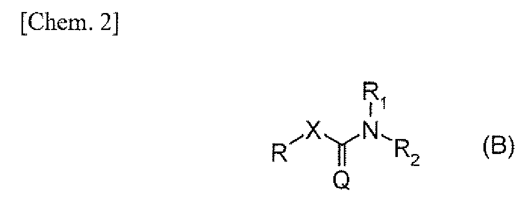 (In the formula, R means various ring groups which may be substituted, or the like, and X and Q are each the same as or different from each other and represent O or S. Further, R₁ and R₂ may form a substituted or unsubstituted ring with the N atom to which they bind to. For details, refer to the publication.)

Furthermore, Patent Document 3 discloses a compound represented by the following formula (C), as a compound having an FAAH inhibitory activity and useful for treatment of urinary frequency, urinary incontinence, overactive bladder, pain, or the like. 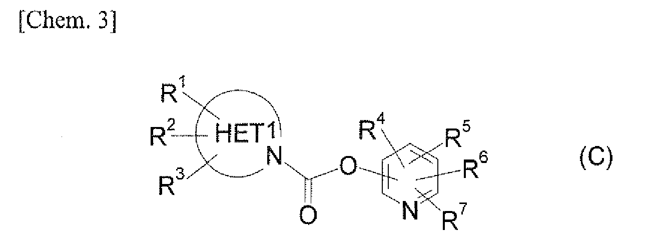 (For details, refer to the publication.)

Furthermore, Patent Document 4 discloses a urea compound represented by the following formula (D), as a compound having an FAAH inhibitory activity and useful for treatment of urinary frequency, urinary incontinence, overactive bladder, or the like. 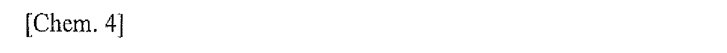 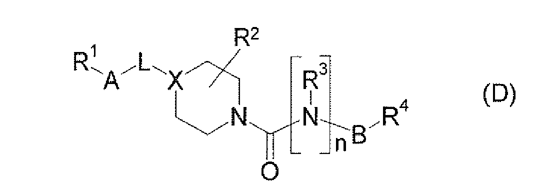 (For details, refer to the publication.)
However, any of the compounds disclosed in these Documents have different structures from that of the compound of the formula (I).

### List of the Documents

### Patent Documents

[Patent Document 1] Pamphlet of International Publication WO2003/065989
[Patent Document 2] Pamphlet of International Publication WO2004/0334
[Patent Document 3] Pamphlet of International Publication W02006/088075
[Patent Document 4] Pamphlet of International Publication WO2008/023720

### Summary of the Invention

### Problem that the Invention is to Solve

A compound useful as an active ingredient of a pharmaceutical composition, particularly a pharmaceutical composition for treating FAAH-related diseases is provided.

### Means for Solving the Problem

The present inventors have extensively studied compounds having an FAAH inhibitory activity, and as a result, have found that the compound of the formula (I) has an excellent FAAH inhibitory activity, and an action to increase the effective bladder capacity, an action to ameliorate sleep disorders, an anti-diuretic action, and an analgesic activity on lower urinary tract pain including bladder pain and the like, based on the inhibitory activity, thereby completed the present invention.
That is, the present invention relates to the follows.
[1] A compound of the formula (I) or a salt thereof: 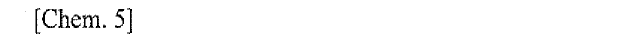 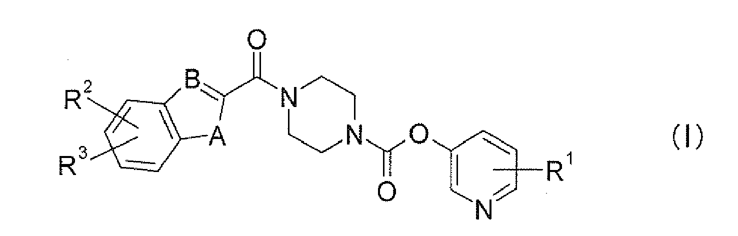 (wherein
   A is NR⁴, S, or O,
   B is CR⁵ or N,
   R¹ is R⁰, halogen, -CO-O-lower alkyl, or -CO-N(R⁰)₂,
   R² and R³ are the same as or different from each other and represent R⁰, -OR⁰, -O-benzyl, halogen, halogeno-lower alkyl, or -CO-aryl,
   R⁴ and R⁵ are the same as or different from each other and represent R⁰, and
   R⁰ are the same as or different from each other and represent H or lower alkyl).
[2] The compound or a salt thereof of [1], wherein the compound is selected from the group consisting of:
   pyridin-3-yl 4-(1,3-benzothiazol-2-ylcarbonyl)plperazine-1-carboxylate,
   pyridin-3-yl 4-[(5,6-dichloro-1H-benzimidazol-2-yl)carbonyl]piperazine-1-carboxylate,
   pyridin-3-yl 4-[(4,5-difluoro-1H-benzimidazol-2-yl)carbonyl]piperazine-1-carboxylate,
   pyridin-3-yl 4-[(3-methyl-1-benzofuran-2-yl)carbonyl]piperazine-1-carboxylate,
   pyridin-3-yl 4-[(4-methyl-1H-benzimidazol-2-yl)carbonyl]piperazine-1-carboxylate,
   pyridin-3-yl 4-(1H-benzimidazol-2-ylcarbonyl)piperazine-1-carboxylate,
   pyridin-3-yl 4-[(1-methyl-1H-indol-2-yl)carbonyl]piperazine-1-carboxylate,
   pyridin-3-yl 4-(1-benzofuran-2-ylcarbonyl)piperazane-1-carboxylate,
   pyridin-3-yl 4-[(5-fluoro-1H-benzimidazol-2-yl)carbonyl]piperazane-1-carboxylate,
   pyridin-3-yl 4-[(5-methyl-1H-benzimidazol-2-yl)carbonyl]piperazine-1-carboxylate,
   pyridin-3-yl 4-[(5,6-dimethyl-1H-benzimidazol-2-yl)carbonyl]piperazine-1-carboxylate, and
   pyridin-3-yl 4-[(5-chloro-1H-benzimidatol-2-yl)carbonyl]piperazine-1-carboxylate.
[3] A pharmaceutical composition comprising the compound or a salt thereof of [1] and a pharmaceutically acceptable excipient.
[4] A pharmaceutical composition for treating nocturia, interstitial cystitis, painful bladder syndrome, or chronic non-bacterial prostatitis/chronic pelvic pain syndrome, comprising the compound or a salt thereof of [1].
[5] Use of the compound or a salt thereof of [1] for the manufacture of a pharmaceutical composition for treating nocturia, interstitial cystitis, painful bladder syndrome, or chronic non-bacterial prostatitis/chronic pelvic pain syndrome.
[6] Use of the compound or a salt thereof of [1] for treating nocturia, interstitial cystitis, painful bladder syndrome, or chronic non-bacterial prostatitis/chronic pelvic pain syndrome.
[7] A method for treating nocturia, interstitial cystitis, painful bladder syndrome, or chronic non-bacterial prostatitis/chronic pelvic pain syndrome, comprising administering to a patient an effective amount of the compound or a salt thereof of [1].

In this connection, unless otherwise specifically noted, when a symbol in a chemical formula in the present specification is also used in another chemical formula, the same symbol has the same meaning.

### Effects of the Invention

The compound of the formula (I) or a salt thereof has an excellent FAAH receptor inhibitory action, and has an action to increase the effective bladder capacity, an action to ameliorate sleep disorders, an anti-diuretic action, and an analgesic activity on lower urinary tract pain including bladder pain and the like, based on the inhibitory action. Thus, it can be used as an agent for preventing and/or treating FAAH-related diseases, particularly nocturia, interstitial cystitis, painful bladder syndrome, or chronic non-bacterial prostatitis/chronic pelvic pain syndrome.
Herein, examples of the FAAH-related diseases include anxiety, depression, epilepsy, brain disorder (head injury, cerebral ischemia, dementia, and the like), urinary frequency, urinary incontinence, overactive bladder, pain, immunological diseases, inflammatory diseases, vomiting, eating disorders, irritable bowel syndrome, ulcerative colitis, hypertension, glaucoma, and the like, in addition to the above-described nocturia, interstitial cystitis, painful bladder syndrome, and chronic non-bacterial prostatitis/chronic pelvic pain syndrome.

### Mode for Carrying out the Invention

Hereinbelow, the present invention will be described in detail.

In the present specification, the "lower alkyl" is linear or branched alkyl having 1 to 6 carbon atoms (hereinafter simply referred to as C₁₋₆), for example methyl, ethyl, n-propyl, isopropyl, n-butyl, isobutyl, sec-butyl, tert-butyl, n-pentyl, n-hexyl, and the like. In another embodiment, it is C₁₋₄ alkyl, and in a further embodiment, methyl or ethyl.

The "halogen" means F, Cl, Br, or I.

The "halogeno-lower alkyl" refers to C₁₋₆ alkyl substituted with one or more halogen atoms. In another embodiment, it is lower alkyl substituted with 1 to 5 halogen atoms, and in a further embodiment, trifluoromethyl.

The "aryl" is a C₆₋₁₄ monocyclic to tricyclic aromatic hydrocarbon ring group, and examples thereof include phenyl and naphthyl, and in a further embodiment, phenyl.

Further, if plurality of substituents present on the same atom as in the case of R⁰ in -CO-N(R⁰)₂, the substituents may be the same as or different from each other.

Embodiments according to the present invention will be presented below.
(1) The compound wherein B is CR⁵ and A is NR⁴ or O. In another embodiment, the compound wherein B is CR⁵ and A is O. In a further embodiment, the compound wherein B is N and A is NR⁴.
(2) The compound wherein R⁴ is H. In another embodiment, the compound wherein R⁴ is methyl.
(3) The compound wherein R⁵ is H. In another embodiment, the compound wherein R⁵ is methyl.
(4) The compound wherein R¹ is H, halogen, or lower alkyl. In another embodiment, the compound wherein R¹ is H, Cl, or methyl. In a further embodiment, the compound wherein R¹ is H or Cl.
(5) The compound wherein R² and R³ are the same as or different from each other and represent H, methyl, F, Cl, Br, trifluoromethyl, or benzyloxy. In another embodiment, the compound wherein R² and R³ are the same as or different from each other and represent H, methyl, F, or Cl.
(6) The compound formed by combination of two or more groups as described in (1) to (5) above.

The compound of the formula (I) may have tautomers or geometrical isomers in some cases, depending on the kind of substituents. In the present specification, the compound of the formula (I) shall be described in only one form of the isomers, yet the present invention includes other isomers, isolated forms of the isomers, or a mixture thereof.
In addition, the compound of the formula (I) may have asymmetric carbon atom(s) or axial asymmetry in some cases, and correspondingly, it may exist in the form of optical isomers. The present invention includes both an isolated form of these optical isomers of the compound of the formula (I) or a mixture thereof.

In addition, pharmaceutically acceptable prodrugs of the compound represented by the formula (I) are also included in the present invention. The pharmaceutically acceptable prodrug refers to a compound having a group which can be converted into amino group, hydroxyl group, carboxyl group, or the like, by solvolysis or under a physiological condition. Examples of the group for forming a prodrug include those as described in Prog. Med., 5, 2157-2161 (1985) or *"*Iyakuhin no Kaihatsu (Pharmaceutical Research and Development)" (Hirokawa Publishing Company, 1990), vol. 7, Bunshi Sekkei (Drug Design), 163-198.

Furthermore, the salt of the compound of the formula (I) is a pharmaceutically acceptable salt of the compound of the formula (I) and may form an acid addition salt or a salt with a base, depending on the kind of substituents. Specifically, examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditolyl tartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like or salts with organic bases such as methylamine, ethylamine, ethanolamine, lysine, ornithine, and the like, salts with various amino acids and amino acid derivatives such as acetylleucine and the like, ammonium salts, and others.

In addition, the present invention also includes various hydrates or solvates, and any of crystalline polymorphs of the compound of the formula (I) and a salt thereof. Also, the present invention includes compounds labeled with various radioactive or nonradioactive isotopes.

### (Production Processes)

The compound of the formula (I) and a salt thereof can be prepared by applying various known synthetic methods, using the characteristics based on their basic skeletons or the kind of substituents. At this time, depending on the type of the functional groups, it is in some cases effective, from the viewpoint of the preparation techniques, to substitute the functional group with an appropriate protective group (a group which is capable of being easily converted into the functional group), during the stage of starting material or intermediate. Examples of the protective group include the protecitve groups described in "Greene's Protective Groups in Organic Synthesis (4th edition, 2006)", written by P. G. M. Wuts and T. W. Greene, and the like, which may be appropriately selected and used depending on reaction conditions. In these methods, a desired compound can be obtained by introducing the protective group to carry out the reaction, and then, if desired, removing the protective group.
In addition, the prodrug of the compound of the formula (I) can be prepared by introducing a specific group during the stage of starting material or intermediate, in the same manner as for the aforementioned protective groups, or by further carrying out the reaction using the obtained compound of the formula (I). The reaction can be carried out by applying a method known to a person skilled in the art, such as common esterification, amidation, dehydration, and the like.
Hereinbelow, the representative production processes for the compound of the formula (I) will be described. Each of the production processes may also be carried out with reference to the References appended in the explanation. Further, the production processes of the present invention are not limited to the examples as shown below.

### (Production Process 1)

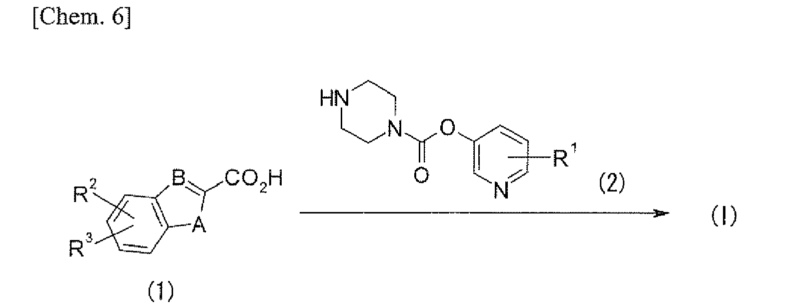

The compound of the formula (I) can be obtained by the reaction of a compound (1) with a compound (2).
This reaction is carried out using the compound (1) and the compound (2) in an equivalent amount or with either thereof in an excess amount, under cooling to heating, preferably at -20°C to 60°C, usually by stirring the mixture thereof for 0.1 hours to 5 days in a solvent which is inert to the reaction, in the presence of a condensing agent. The solvent as used herein is not particularly limited, but examples thereof include aromatic hydrocarbons such as benzene, toluene, xylene, and the like, halogenated hydrocarbons such as dichloromethane, 1,2-dichloroethane, chloroform, and the like, ethers such as diethyl ether, tetrahydrofuran (THF), dioxane, dimethoxyethane (DME), and the like, N,N-dimethylformamide (DMF), dimethylsulfoxide (DMSO), ethyl acetate (EtOAc), acetonitrile, water, or a mixture thereof. Examples of the condensing agent include 1-(3-dimethylaminopropyl)-3-ethylcarbodiimide (WSC), dicyclohexylcarbodiimide (DCC), 1,1'-carbonyldiimidazole (CDI), diphenylphosphoryl azide (DPPA), phosphorus oxychloride, and the like, but are not limited thereto. It may be preferable in some cases for the reaction to use an additive (for example, 1-hydroxybenzotriazole (HOBt) and the like). It may be advantageous in some cases for the smooth progress of the reaction to carry out the reaction in the presence of an organic base such as triethylamine, N,N-diisopropylethylamine, N-methylmorpholine, and the like, or an inorganic base such as potassium carbonate, sodium carbonate, potassium hydroxide, and the like.
Furthermore, a method in which the carboxylic acid (1) is converted into a reactive derivative thereof, and then reacted with the amine (2) can also be used. Here, examples of the reactive derivative of the carboxylic acid include acid halides obtained by the reaction with a halogenating agent such as phosphorus oxychloride, thionyl chloride, and the like, mixed acid anhydrides obtained by the reaction with isobutyl chloroformate or the like, active esters obtained by condensation with HOBt, or the like, and others. The reaction of the reactive derivative and the compound (2) can be carried out under cooling to heating, preferably at -20°C to 60°C in a solvent which is inert to the reaction, such as halogenated hydrocarbons, aromatic hydrocarbons, ethers, and the like.
[References]
"Organic Functional Group Preparations", written by S. R. Sandler and W. Karo, 2nd edition, Vol. 1, Academic Press Inc., 1991
"Courses in Experimental Chemistry (5th edition)", edited by The Chemical Society of Japan, Vol. 16 (2005) (Maruzen)

### (Production Process 2)

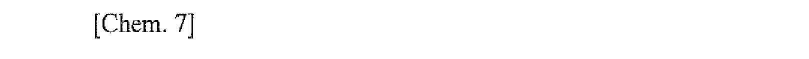

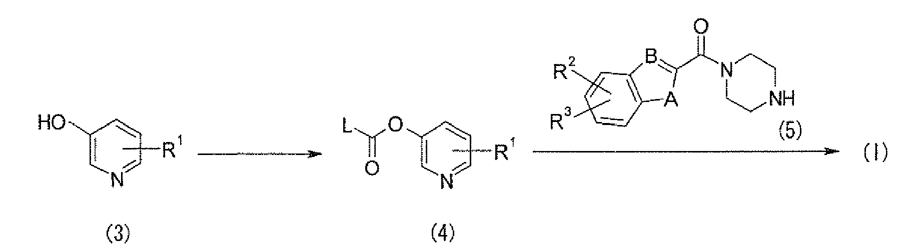

(In the formula, L represents a leaving group.)
The compound of the formula (I) can be obtained by converting a compound (3) into a carbonic ester derivative (4) thereof, and then reacting with an amine compound (5).
Here, examples of the leaving group include Cl, 1-imidazolyl, phenoxy, and 4-nitrophenoxy groups.
The first step is carried out by reacting the compound (3) with a carbonylating reagent in an equivalent amount or in an excess amount, under cooling to heating, preferably at -20°C to 80°C, usually for about 0.1 hour to 1 day, in a solvent which is inert to the reaction, in the presence of a base. In the next step, while the reaction of the first step is not treated, to the reaction mixture is added the amine compound (5) in an equivalent amount or in an excess amount, and the mixture is reacted under cooling to heating, preferably at -20°C to 80°C, usually for about 0.1 hour to 1 day. The solvent as used herein is not particularly limited, but examples thereof include halogenated hydrocarbons such as dichloromethane, 1,2-dichloroethane, chloroform, and the like, aromatic hydrocarbons such as benzene, toluene, xylene, and the like, ethers such as diethylether, THF, dioxane, DME, and the like, DMF, DMSO, EtOAc, acetonitrile, or a mixture thereof. Examples of the carbonylating reagent include diphosgene, triphosgene, CDI, 4-nitrophenyl chloroformate, and phenyl chloroformate. When the carbonic ester derivative (4) which is an intermediate is stable, this may be first isolated and then the next reaction may be carried out.
[Reference]
"Organic Functional Group Preparations", written by S. R. Sandler and W. Karo, 2nd edition, Vol. 2, Academic Press Inc., 1991

### (Starting Material Synthesis 1)

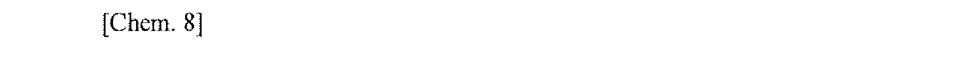

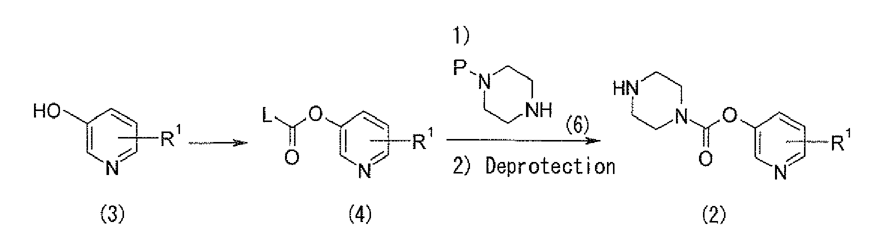

(In the formula, P represents a protective group for the amino group, for example, is benzyloxycarbonyl and tert-butoxycarbonyl (Boc).)
The compound (2) can be prepared by reacting the compound (3) with an appropriately protected piperazine (5) according to Production Process 2 as described above, and then removing the protective group for the amino group.

### (Starting Material Synthesis 2)

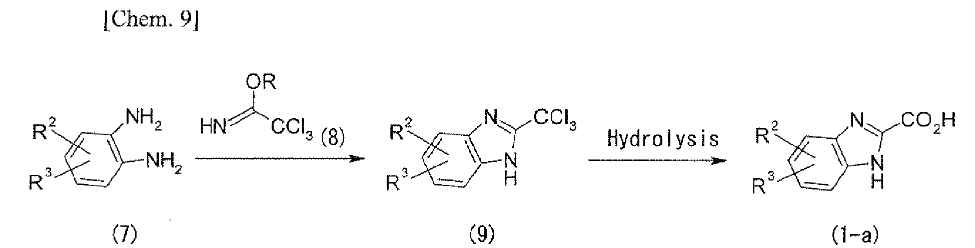

(In the formula, R represents lower alkyl, and preferably methyl or ethyl.)
A compound (1-a) wherein A is NH and B is N in the formula (1) can be prepared by reacting a compound (7) with a compound (8) in acetic acid at room temperature to under heating to construct a benzimidazole ring, and then hydrolyzing the trichloromethyl group at the 2-position in benzimidazole under basic conditions.
[Reference]
Eur. J. Med. Chem., Vol. 28, pp. 71-75, 1933
(Starting Material Synthesis 3) 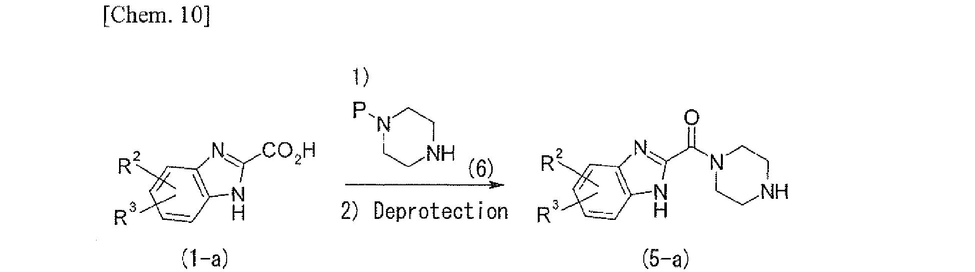 The compound (5-a) wherein A is NH and B is N in the formula (5) can be prepared by reacting the compound (1) with an appropriately protected piperazine (6) according to Production Process 1 as described above, and then removing the protective group for the amino group.

The compound of the formula (I) is isolated and purified as its free compounds, salts, hydrates, solvates, or crystalline polymorphorous substances. The salt of the compound of the formula (I) can also be prepared in accordance with a conventional method for a salt formation reaction.
Isolation and purification are carried out by employing general chemical operations such as extraction, fractional crystallization, various types of fractional chromatography, and the like.
Various isomers can be prepared by selecting an appropriate starting material or isolated by making use of the difference in the physicochemical properties between isomers. For example, optical isomers can be obtained by means of general optical resolution methods of racemic compounds (for example, fractional crystallization for inducing diastereomer salts with optically active bases or acids, chromatography using a chiral column, and the like). In addition, the isomers can also be prepared from a suitable optically active starting material.

The pharmacological activities of the compound of the formula (I) were confirmed by the following tests.
Test Example 1: Screening for an FAAH activity-inhibiting substance using a human bladder epithelial cancer-derived cell
(1) Screening for an FAAH activity-inhibiting substance
   Human bladder epithelial cancer-derived cell line 5637 cells (HTB-9; ATCC) were seeded on a 48-well cell culture plate in an amount of 1 ×10⁵ cell/well, using 10% fetal bovine serum (Hyclone Laboratories, Inc.)-containing RPMI1640 medium (Invitrogen Corporation). After being incubated at 37°C for 12 hours or longer, the cells were washed with 400 µl/well of a buffer (Hank's Balanced Salt Solution, 20 mM Hepes-NaOH (pH 7.4)). A test substance dissolved in DMSO was added to a substrate solution (the above buffer containing 3 µCi/ml radiolabeled anandamide (Anandamide [ethanolamine 1-³H]) and 10 µM anandamide) so as to have a concentration from 0.003 nM to 30 nM. As a control, DMSO alone was added. 100 µl/well of the substrate solution was added to the above cells, and incubated in a CO₂ incubator at 37°C for 30 minutes. Next, the cell culture plate was transferred onto ice, and the substrate solution was removed by suction; and 75 µl/well of a cytolytic solution that had been ice-cooled (the above buffer containing 0.5% Triton X-100, and 10 µM of a compound having an FAAH-inhibitory activity, cyclohexylcarbamic acid 3'-carbamoylbiphenyl-3-yl ester (URB597; Cayman chemical; Kathuria et al., Nature Med., Vol. 9, pp. 76-81, 2003)) was added thereto, followed by stirring. The resulting cell lysate in wells were individually transferred into a 1.5 ml sample tube, to which 150 µl of 1:1 (ratio by volume) chloroform/methanol solution was added, followed by stirring. This was centrifuged (15000 revolutions/minute, 2 minutes), whereby the decomposed product, ethanolamine (ethanolamine 1-³H) was separated in the upper layer (water/methanol layer) and the unreacted radiolabeled anandamide was in the lower layer (chloroform layer). 25 µl of the upper layer was transferred into a 96-well organic solvent-resistant white microplate (PicoPlate-96; PerkinElmer, Inc.), 150 µl of Microscint-20 (PerkinElmer, Inc.) was added thereto, and this was measured with a microplate scintillation counter (TopCount^{™}; Beckman Coulter, Inc.). As compared with the control, the substance that gave a decreased value was selected as an FAAH activity-inhibiting substance.
(2) Measurement of the IC₅₀ value of the FAAH activity-inhibiting substance
   The compound dissolved in DMSO to have a concentration of 10 mM was added to the substrate solution so as to have a concentration from 0.003 nM to 30 nM. According to the method mentioned above, the compound was analyzed for its influence on FAAH activity. As a negative control, DMSO, and as a positive control, a compound described in Patent Document 2, URB597, were added to the substrate solution to have a concentration of 10 µM. Based on the measured value of the positive control, 0%, and on the measured value of the negative control, 100%, the IC₅₀ value was obtained.

Test Example 2: Screening for an FAAH activity-inhibiting substance using a rat tissue homogenate administered with a test substance
(1) Administration to a rat and preparation of a tissue homogenate
   A test substance suspended in 0.5% methyl cellulose (MC) solution was orally administered to two 9-week-old Wistar male rats (Japan SLC, Inc.) at a dose of 1 mg/kg. As a control, 0.5% MC solution was orally administered to two rats. After 30 minutes, peripheral blood was collected from the rat under ether anesthesia through its abdomen aorta after laparotomy. After that, the head of each rat was cut off, and its right cerebrum was taken out.
   To the collected rat brain was added 2 ml of an ice-cooled buffer (50 mM Tris-HCl (pH 8.0), 1 mM EDTA, 0.32 M sucrose), followed by homogenization with a homogenizer in ice to give a uniform suspension. Further, using an ultrasonic wave generator (UR-20P (Power dial 4), Tomy Seiko Co., Ltd.), this was ultrasonicated for 5 seconds. The protein concentration of the obtained homogenates was measured according to a dye-coupling method (protein assay CBB solution, Nacalai Tesque, Inc.). Using a buffer (50 mM Tris-HCl (pH 8.0), 1 mM EDTA), the homogenates of the rat brain were diluted so that their protein concentration was 80 µg/ml, thereby giving enzyme solutions.
   In addition, to the collected peripheral blood was added an equivalent amount of physiological saline for dilution. 3 ml of Lymphosepar II (Immuno-Biological Laboratories) was put into a 15 ml centrifuge tube and 3 ml of the diluted peripheral blood was added thereto quietly. After centrifugation (2000 revolutions/min, 20°C, 20 minutes), a monocyte layer was collected and washed twice with physiological saline. After centrifugation, to the obtained monocytes, 200 µl of an ice-cooled buffer (50 mM Tris-HCl (pH 8.0), 1 mM EDTA) was added, and using an ultrasonic wave generator (UR-20P (power dial 4), Tomy Seiko Co., Ltd.), this was ultrasonicated for 5 seconds. The protein concentration of the obtained homogenates was measured according to a dye-coupling method (protein assay CBB solution, Nacalai Tesque, Inc.). Using a buffer (50 mM Tris-HCl (pH 8.0), 1 mM EDTA), the homogenates of rat monocytes were diluted so that their protein concentration was 400 µg/ml, thereby preparing enzyme solutions.
(2) Measurement of FAAH activity
   To 50 µl of the enzyme solution was added 50 µl of a substrate solution (74 kBq/ml radiolabeled anandamide (Anandamide [ethanolamine 1-³H] (Muromachi Technos Co., Ltd.)), 50 mM Tris-HCl (pH 8.0), 1 mM EDTA), followed by reaction at room temperature for 60 minutes. 200 µl of a 1:1 (ratio by volume) solution of chloroform and methanol was added thereto, followed by stirring and centrifuging (15000 revolutions/minute, 2 minutes). 30 µl of the upper layer was transferred into a 96-well organic solvent-resistant white microplate (PicoPlate-96; PerkinElmer, Inc.), 150 µl of Microscinti-20 (PerkinElmer, Inc.) was added thereto, followed by measurement with a microplate scintillation counter (TopCount^{™}; Beckman Coulter, Inc.).
   By taking the FAAH activity of the control rat not administered with the test substance as 100% and taking the FAAH activity of the tissue homogenate-free buffer (50 mM Tris-HCl (pH 8.0), 1 mM EDTA) as 0%, the relative value (%) of the FAAH activity of the rat tissue homogenate administered with the test substance was determined. The inhibitory rate (%) was calculated by subtracting the obtained relative value (%) from 100%.

For several compounds of the formula (I), the results (IC₅₀ values) of Test Example 1 above and the results (inhibitory rates) of Test Example 2 are shown in the Table. Further, in the Table, Ex represents the Example No. as denoted below and- represents no evaluation.

**[Table 1]**

| Compound | IC₅₀ (nM) | Inhibitory rate (%) | |
|---|---|---|---|
| | | Monocytes | Brain |
| Ex 8 | 0.13 | - | - |
| Ex 13 | 0.13 | - | - |
| Ex 18 | 0.98 | 80 | 92 |
| Ex 20 | 0.28 | 78 | 87 |
| Ex 23 | 0.82 | 88 | 94 |
| Comparative Compound A | 0.28 | 7 | 5 |
| Comparative Compound B | 2.3 | 0 | 0 |
| Comparative Compound C | 3.7 | 6 | 7 |
| Comparative Compound D | - | 42 | 10 |

| | | | |
|---|---|---|---|
| Comparative Compound A: Compound of Example 137 in Patent Document 3 Comparative Compound B: Compound of Example 169 in Patent Document 4 Comparative Compound C: Compound of Example 351 in Patent Document 4 Comparative Compound D: Compound of Example 361 in Patent Document 4 | | | |

Test Example 3: Action of the compound on rats having cyclophosphamide (CPA)-induced urinary frequency
Disease models were used to investigate an action to improve the bladder irritability symptom of the compound. Cyclophosphamide (CPA) is known to be converted to the metabolite acrolein by systemic administration and to damage the bladder mucosa via the urine. In rats, since CPA administration induces bladder pain or urinary frequency conditions associated with hemorrhagic cystitis, the drug efficacy on these symptoms can be evaluated. For the experiment, 9-week-old Wistar female rats (Charles River Laboratories Japan, Inc.) were used. CPA (100 mg/kg) was administered intraperitoneally, and after 2 days, the experiment was carried out. The compound was orally administered and after 15 minutes, distilled water (30 ml/kg) was forcibly orally administered. The rats were confined in a metabolic cage and the urine weight was continuously measured for 1 hour. A total urine weight was divided by the total urination frequency to determine an effective bladder capacity. As a result, in the group administered with 0.5% methylcellulose which is a solvent, the bladder capacity decreased and the urinary frequency condition was found. The effective oral dose of a certain compound of the formula (I) was 1 mg/kg, and the compounds increased the reduced effective bladder capacity and improved the urinary frequency condition.

Test Example 4: Action of the compound on water-loaded rats (anti-diuretic action test)
Water-loaded rats were used to investigate an action to decrease the urine amount by the compound. By forcing distilled water to be orally administered, the urine amount increases, and thus, the drug efficacy on the urine amount can be evaluated. For the experiment, 9- to 11-week-old Wistar male rats (Japan SLC, Inc.) were used. The compound was orally administered, and after 30 minutes, distilled water (30 ml/kg) was forcibly orally administered. The rats were confined in a metabolic cage and the urine weight was continuously measured for 6 hours. The compounds of Examples 18, 20, and 23 were effective at a dose of 10 mg/kg or less, and as compared with the group administered with 0.5% methylcellulose which is a solvent, the urine amounts were decreased.

Test Example 5: Action of the compound on rats having sleep disturbance conditions (Test on an action to ameliorate sleep disorders)
Brain wave electrode-implanted rats were used to investigate the action to ameliorate sleep disorders by the compound. Grids were installed at a position of 7 cm from a bottom of an acrylic cylindrical cage having a diameter of 30 cm and a height of 50 cm at an interval of 2 cm, and water was filled under the grids. Waking hours increase due to sleep disturbance by placing the rats on the grids, and thus, the drug efficacy for sleep disturbance can be evaluated. For the experiment, 10- to 12-week-old Wistar male rats (Charles River Laboratories Japan, Inc.) were used. The compound was orally administered, and immediately after administration, the spontaneous brain waves and electromyogram were measured continuously for 6 hours. The effective oral dose of a certain compound of the formula (I) was 3 mg/kg, and in the group administered with 0.5% methylcellulose which is a solvent, the increased waking hours decreased.

As a result of the above tests, it was confirmed that the compound of the formula (I) has an excellent FAAH inhibitory action, and has an action to increase the effective bladder capacity and an action to ameliorate sleep disorders, as well as an anti-diuretic action. Accordingly, the compound can be used to treat FAAH-related diseases, particularly nocturia, or the like.

Interstitial cystitis, painful bladder syndrome, and chronic non-bacterial prostatitis/chronic pelvic pain syndrome are all diseases having urinary frequency and bladder pain as one of the main symptoms (Neurourology and Urodynamics, Vol. 21, pp. 167-178, 2002; The Journal of Urology, Vol. 168, pp. 593-598, 2002). Further, it is known that 40 to 50% of patients with chronic non-bacterial prostatitis/chronic pelvic pain syndrome involve testicular pain (The Journal of Urology, Vol. 168, pp. 593-598, 2002; The Journal of Urology, Vol. 162, pp. 369-375, 1999). Accordingly, the experiments below were also carried out.

Test Example 6: Action of the compound on bladder pain model rats
Disease models were used to investigate the analgesic action of the compound on bladder pain. Cyclophosphamide (150 mg/kg) was administered intraperitoneally, and after 2 days, physiological saline was infused at a flow rate of 45 ml/h through a cannula transurethrally inserted into the bladder under unrestrained condition to extend the bladder rapidly. By rapidly distending the bladder, amplification of external oblique electromyogram spikes accompanied by pain-related behavior is found. Since an injection amount at that time can be defined as a bladder pain threshold, drug efficacy can be evaluated for the bladder pain threshold. For the experiment, 7-week-old SD female rats (Charles River Laboratories Japan, Inc.) were used. The compound was orally administered to the rats pre-treated with cyclophosphamide, and after 60 minutes, the bladder pain thresholds were measured. As a result, in the group administered with 0.5% methylcellulose which is a solvent, the bladder pain threshold was decreased, and thus, it was found that bladder pain was elicited. The effective oral dose of a certain compound of the formula (I) was 3 mg/kg, and the compound increased the reduced bladder pain threshold and showed an analgesic action on bladder pain.

Test Example 7: Action of the compound on testicular pain model rats
Disease models were used to investigate the analgesic action of the compound on testicular pain. When 1% acetic acid is administered to the left and right testes of the rats at 1 ml/kg each, pain behavior associated with testicular pain is observed, and drug efficacy on the pain behavior can be evaluated. For the experiment, 3- to 4-week-old Wistar male rats (Charles River Laboratories Japan, Inc.) were used. The compound was orally administered to the rats, and after 55 minutes, acetic acid was administered into the testicle, and the rats were transferred to acrylic cylindrical cages having a diameter of 30 cm and a height of 50 cm. From 5 minutes after administration of acetic acid, the frequency of pain behavior was measured for 15 minutes. The effective oral dose of a certain compound of the formula (I) was 1 mg/kg, and as compared with the group administered with 0.5% methylcellulose which is a solvent, pain behavior decreased significantly, showing an analgesic action on testicular pain.

From the results of Test Examples 3, 6, and 7 above, it was confirmed that the compound of the formula (I) increases the effective bladder capacity in the urinary frequency models and has an analgesic action in bladder pain model and testicular pain model. Accordingly, it can be used for treatment of interstitial cystitis, painful bladder syndrome, chronic non-bacterial prostatitis/chronic pelvic pain syndrome, and the like.

Moreover, for the purpose of confirming the effect of the compound of the formula (I) on pain, evaluation was carried out using L5/L6 spinal nerve ligation rats that are neuropathic pain models (see Patent Document 3). As a result, the effect of a certain compound of the formula (I) on pain was confirmed. Accordingly, the compound of the formula (I) can be used also for treatment of pain, and the like.
Herein, the pain is a generic term for neuropathic pain, nociceptive pain, inflammatory pain, and the like. Among these, neuropathic pain refers to pain due to peripheral or central nervous system dysfunction, and includes pain associated with diabetic neuropathy, cancer pain, trigeminal neuralgia, phantom limb pain, postherpetic neuralgia, thalamic pain, and the like. Further, examples of inflammatory pain include, but are not limited to, pain associated with osteoarthritis. It is thought that the compound of the formula (I) is effective particularly for neuropathic pain.

A pharmaceutical composition containing one or two or more kinds of the compound of the formula (I) or a salt thereof as an active ingredient can be prepared in accordance with a generally used method, using an excipient, that is, a pharmaceutical excipient, a pharmaceutical carrier, or the like, that is usually used in the art.
The administration can be carried out through any mode of oral administration via tablets, pills, capsules, granules, powders, liquid preparations, or the like, or parenteral administration via injections such as intraarticular, intravenous, intramuscular, or others, suppositories, eye drops, eye ointments, percutaneous liquid preparations, ointments, percutaneous patches, transmucosal liquid preparations, transmucosal patches, inhalations, and the like.

Regarding solid composition for oral administration, tablets, powders, granules, or the like are used. In such a solid composition, one or two or more active ingredients are mixed with at least one inactive excipient such as lactose, mannitol, glucose, hydroxypropylcellulose, microcrystalline cellulose, starch, polyvinyl pyrrolidone, magnesium aluminometasilicate, and/or the like. According to a conventional method, the composition may contain inactive additives such as lubricants such as magnesium stearate and the like, disintegrators such as sodium carboxymethyl starch and the like, stabilizers, and solubilizing agents. Tablets or pills may be coated with sugar coating, or with a film of gastric or enteric substance if necessary.
The liquid composition for oral administration includes pharmaceutically acceptable emulsions, solutions, suspensions, syrups, elixirs, or the like, and contains a generally used inert diluent, such as purified water or ethanol. In addition to the inert diluent, the liquid composition may contain adjuvants such as solubilizing agents, moistening agents, and suspending agents, sweeteners, flavors, aromatics, and antiseptics.

Injections for parenteral administration include sterile, aqueous or non-aqueous solutions, suspensions, or emulsions. As the aqueous solvent, for example, distilled water for injection or physiological saline is included. Examples of the non-aqueous solvent include propylene glycol, polyethylene glycol, vegetable oils such as olive oil and the like, alcohols such as ethanol and the like, Polysorbate 80 (Pharmacopeia), etc. Such a composition may further contain tonicity agents, antiseptics, moistening agents, emulsifying agents, dispersing agents, stabilizers, or solubilizing agents. These are sterilized, for example, by filtration through a bacteria-retaining filter, blending with bactericides, or irradiation. In addition, these can also be used by producing sterile solid compositions, and dissolving or suspending it in sterile water or a sterile solvent for injection prior to their use.

The agent for external use includes ointments, plasters, creams, jellies, cataplasms, sprays, lotions, eye drops, eye ointments, and the like. The agents contain generally used ointment bases, lotion bases, aqueous or non-aqueous liquid preparations, suspensions, emulsions, and the like. Examples of the ointment bases or the lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, sorbitan sesquioleate, and the like.

Regarding transmucosal agents such as inhalations, transnasal agents, and the like, agents in solid, liquid or semi-solid state are used, and they can be prepared in accordance with conventionally known methods. For example, known excipients, as well as pH adjusting agents, antiseptics, surfactants, lubricants, stabilizers, thickeners, or the like may be appropriately added thereto. For their administration, appropriate devices for inhalation or insufflation may be used. For example, a compound may be administered alone or as powders of formulated mixture, or as solution or suspension by combining it with pharmaceutically acceptable carriers, using conventionally known devices or sprayers, such as a measured administration inhalation device and the like. The dry powder inhalers or the like may be for single or multiple administration use, and dry powders or powder-containing capsules may be used. Alternatively, this may be in a form of a pressurized aerosol spray which uses an appropriate propellant such as chlorofluoroalkane or hydrofluoroalkane, or a suitable gas such as carbon dioxide, or the like.

In the case of oral administration, it is appropriate that the daily dose may be usually from about 0.001 to 100 mg/kg, preferably from 0.1 to 30 mg/kg, and more preferably 0.1 to 10 mg/kg per body weight, and this is administered in a single portion or divided into 2 to 4 portions. Also, in the case of intravenous administration, the appropriate daily dose is from about 0.0001 to 10 mg/kg per body weight, and administration is made once a day or two or more times a day. In addition, a transmucosal agent is administered at a dose from about 0.001 to 100 mg/kg per body weight, once a day or two or more times a day. The dose is appropriately determined in response to an individual case by taking the symptoms, age, and sex, and the like into consideration.

The compound of the formula (I) can be used in combination with various therapeutic agents or prophylactic agents for the diseases, in which the compound of the formula (I) is considered effective, as described above. The combined preparation may be administered simultaneously; or separately, and continuously or at a desired time interval. The preparations to be co-administered may be a blend, or prepared individually.

### Examples

Hereinbelow, the production processes for the compound of the formula (I) will be described in more detail with reference to Examples. In this connection, the present invention is not limited to the compounds described in Examples below. Also, the production processes for the starting material compounds are shown in Preparation Examples. Further, the production processes for the compound of the formula (I) are not limited to the production processes of the specific Examples shown below, and the compound of the formula (I) can be prepared in accordance with a combination of such production processes, or methods apparent to those skilled in the art.

In addition, the following abbreviations may be used sometimes in Examples, Preparation Examples and Tables to be described later.
Pre: Preparation Example No., Ex: Example No., Cpd: Compound No., Str: Structural formula, Syn: Production process (In Examples/Preparation Examples above, the Preparation Example No. or Example No., which was produced in the same manner, is indicated. Here, P represents Preparation Example and E represents Example. For example, it is represented that the compound of Preparation Example 6 was prepared in the same manner as for the compound of Preparation Example 1, and the compound of Example 3 was prepared in the same manner as for the compound of Example 1.), Dat: Physicochemical data (NMR1: δ (ppm) in ¹H NMR in DMSO-d₆, FAB+: FAB-MS (cation), ESI+: ESI-MS (cation)), mp: melting point (Round brackets show a solvent for recrystallization.), Me: Methyl, Et: Ethyl, Bn: Benzyl, Boc: tert-Butoxycarbonyl, iPr₂O: Diisopropyl ether, MeOH: Methanol, Hex: Hexane, EtOH: Ethanol, TFA: Trifluoroacetic acid.

### Preparation Example 1

To a solution of methyl 2,2,2-trichloroacetimidate (1.53 g) in acetic acid (10 ml) was added a solution of 4-fluorobenzene-1,2-diamine in acetic acid (10 ml) under ice-cooling, followed by stirring at room temperature for 3 hours. The reaction liquid was concentrated under reduced pressure and to the obtained residue was added water, followed by neutralization with a saturated aqueous sodium hydrogen carbonate solution and extraction with EtOAc. The organic layer was washed with saturated brine, then dried over anhydrous magnesium sulfate, and concentrated under reduced pressure. The residue was recrystallized from iPr₂O/EtOAc/MeOH to obtain 5-fluoro-2-(trichloromethyl)-benzimidazole (1.5 g) as a beige powder. To a I M aqueous sodium hydroxide solution (30 ml) was added 5-fluoro-2-(trichloromethyl)-benzimidazole (500 mg) under ice-cooling, followed by stirring for 1 hour. The reaction liquid was acidified by the addition of 1 M hydrochloric acid. The resulting solid was collected by filtration and dried under reduced pressure to obtain 5-fluorobenzimidazole-2-carboxylic acid (350 mg) as a beige powder.

### Preparation Example 2

A mixture of tert-butyl-piperazine-1-carboxylate (1.75 g), benzimidazole-2-carboxylic acid (1.69 g), HOBt (1.52 g), WSC (1.75 g), and DMF (35 ml) was stirred at room temperature overnight. To the reaction liquid was added a saturated aqueous sodium hydrogen carbonate solution, followed by extraction with EtOAc. The organic layer was washed with saturated brine, then dried over anhydrous magnesium sulfate, and concentrated under reduced pressure. The residue was purified by silica gel column chromatography (eluent; Hex:EtOAc=2:1 (V/V)) to obtain tert-butyl-4-(benzimidazol-2-ylcarbonyl)piperazine-1-carboxylate (2.4 g) as a white powder.

### Preparation Example 3

To a mixture of tert-butyl-4-(benzimidazol-2-ylcarbonyl)piperazine-1-carboxylate (2.4 g), EtOAc (48 ml), and MeOH (12 ml) was added a 4 M hydrogen chloride/EtOAc solution (9 ml), followed by stirring at room temperature for 5 hours. The resulting solid was collected by filtration, then washed with EtOAc, and dried under reduced pressure to obtain 2-(piperazin-1-ylcarbonyl)-benzimidazole hydrochloride (1.9 g) as a white powder.

### Preparation Example 4

To a suspension of 3-hydroxypyridine (3.62 g) in acetonitrile (160 ml) was added CDI (6.18 g), followed by stirring at room temperature for 1 hour. To the reaction liquid was added a mixture of benzyl-piperazine-1-carboxylate (8.00 g), a 4 M hydrogen chloride/dioxane solution (18.2 ml), and acetonitrile (100 ml) under ice-cooling, followed by stirring at room temperature overnight. To the reaction liquid was added a saturated aqueous sodium hydrogen carbonate solution, followed by extraction with EtOAc. The organic layer was washed with saturated brine, then dried over anhydrous magnesium sulfate, and concentrated under reduced pressure. The residue was purified by silica gel column chromatography (eluent; Hex:EtOAc=1:1 to 1:4 (V/V)) to obtain a colorless oily substance. The obtained oily substance was left to stand overnight, and the resulting solid was washed with iPr₂O and dried under reduced pressure to obtain benzyl-pyridin-3-ylpiperazine-1,4-dicarboxylate (10.5 g) as a white powder.

### Preparation Example 5

To a solution of benzyl-pyridin-3-ylpiperazine-1,4-dicarboxylate (6.0 g) in EtOH (180 ml) was added 5% palladium on carbon (1.87 g), followed by stirring for 4 hours under a hydrogen atmosphere. The catalyst was removed by filtration and the filtrate was concentrated under reduced pressure. The obtained oily substance was dissolved in 1 M hydrochloric acid and washed with EtOAc. The aqueous layer was alkalified with a saturated aqueous sodium hydrogen carbonate solution and extracted with chloroform. The organic layer was dried over anhydrous magnesium sulfate and then concentrated under reduced pressure to obtain a colorless oily substance. The obtained oily substance was dissolved in EtOAc, followed by addition with a 4 M hydrogen chloride/EtOAc solution (8.8 ml) and stirring at room temperature. The resulting solid was collected by filtration and dried under reduced pressure to obtain pyridin-3-ylpiperazine-1-carboxylate hydrochloride (4.2 g) as a white powder.

In the same manner as in the methods of Preparation Examples 1 to 5, the compounds of Preparation Examples 6 to 12 as shown in the Tables below were prepared. The structures, the physicochemical data, and the production processes of the compounds of the Preparation Examples are shown in Tables 2 and 3.

### Example 1

To a mixture of pyridin-3-ylpiperazine-1-carboxylate hydrochloride (250 mg), benzimidazole-2-carboxylic acid (176 mg), HOBt (144 mg), WSC (166 mg), and DMF (5 ml) was added triethylamine (0.25 ml), followed by stirring at room temperature overnight. To the reaction liquid was added a saturated aqueous sodium hydrogen carbonate solution, followed by extraction with EtOAc. The organic layer was washed with saturated brine, dried over anhydrous magnesium sulfate, and then concentrated under reduced pressure. The residue was purified by silica gel column chromatography (eluent; Hex:EtOAc=1:2 (V/V)), and then solidified from Hex/EtOAc, and the obtained solid was dried under reduced pressure to obtain pyridin-3-yl-4-(benzimidazol-2-ylcarbonyl)piperazine-1-carboxylate (245 mg) as a white powder.

### Example 2

To a solution of methyl 5-hydroxynicotinate (113 mg) in DMSO (10 ml) was added CDI (120 mg), followed by stirring at room temperature for 1 hour. To the reaction liquid were added 2-(piperazin-1-ylcarbonyl)-benzimidazole hydrochloride (180 mg) and TFA (0.07 ml), followed by stirring at room temperature overnight. To the reaction liquid was added a saturated aqueous sodium hydrogen carbonate solution, followed by extraction with EtOAc. The organic layer was washed with saturated brine, then dried over anhydrous magnesium sulfate, and concentrated under reduced pressure. The residue was purified by silica gel column chromatography (eluent; Hex:EtOAc=1:2 (V/V)) to obtain 5-(methoxycarbonyl)pyridin-3-yl-4-(benzimidazol-2-ylcarbonyl)piperazine-1-carboxylate (105 mg) as a white powder.

In the same manner as in the methods of Examples 1 and 2, the compounds of Examples 3 to 27 as shown in the Tables below were prepared. The structures, the physicochemical data, and the production processes of the compounds of Examples are shown in Tables 4 to 7. Further, NMR data and mp of several Example Compounds are shown in Tables 8 and 9.

Furthermore, the structures of other compounds among the compounds of the formula (I) are shown in Tables 10 to 16. These can be easily prepared by the methods described in production processes and Examples as described above, methods apparent to a skilled person in the art, or modified methods thereof.

**[Table 2]**

| Pre | Syn | Str | Dat |
|---|---|---|---|
| 1 | P1 | 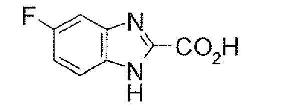 | FAB+: 181 |
| 2 | P2 | 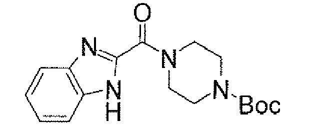 | ESI+: 331 |
| 3 | P3 | 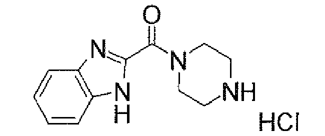 | ESI+: 231 |
| 4 | P4 | 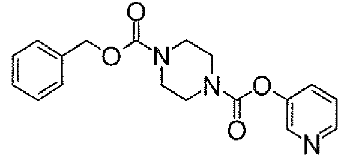 | ESI+: 342 |
| 5 | P5 | 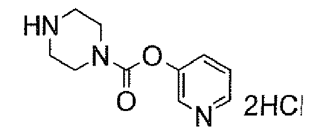 | ESI+: 208 |
| 6 | P1 | 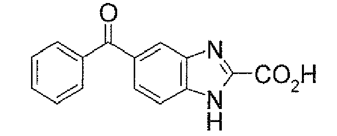 | ESI+: 267 |
| 7 | P1 | 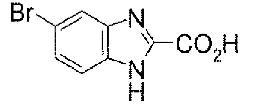 | ESI+: 241 |
| 8 | P1 | 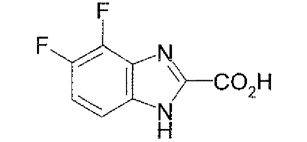 | ESI+: 199 |
| 9 | P1 | 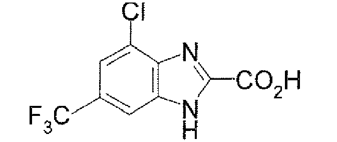 | ESI+: 264 |

**[Table 3]**

| | | | |
|---|---|---|---|
| 10 | P1 | 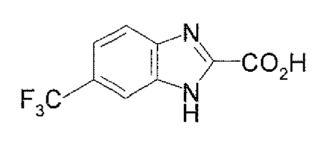 | ESI+: 231 |
| 11 | P2 | 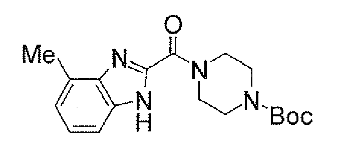 | ESI+: 345 |
| 12 | P3 | 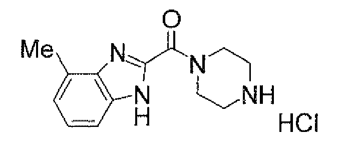 | ESI+: 245 |

**[Table 4]**

| Ex | Syn | Str | Dat |
|---|---|---|---|
| 1 | E1 | 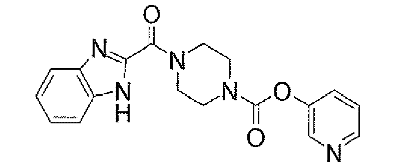 | EST+: 352 |
| 2 | E2 | 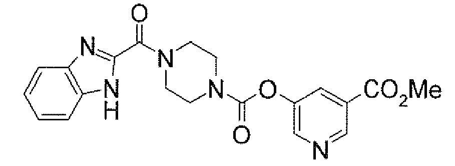 | ESI+: 410 |
| 3 | E1 | 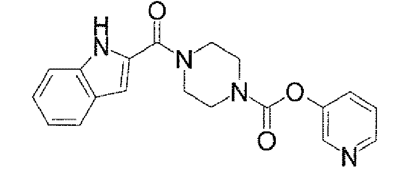 | ESI+: 351 |
| 4 | E1 | 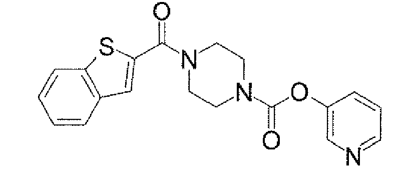 | ESI+: 368 |
| 5 | E1 | 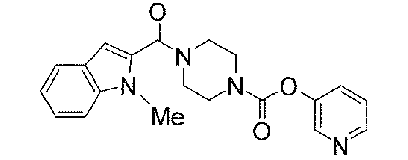 | ESI+: 365 |
| 6 | E1 | 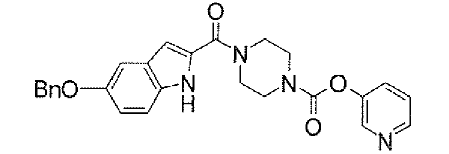 | ESI+: 457 |
| 7 | E1 | 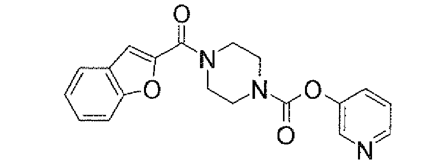 | ESI+: 352 |

**[Table 5]**

| | | | |
|---|---|---|---|
| 8 | E1 | 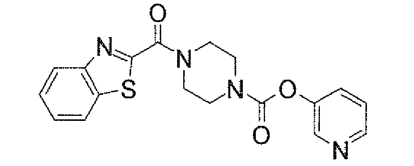 | ESI+: 369 |
| 9 | E1 | 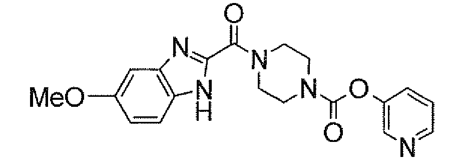 | ESI+: 382 |
| 10 | E1 | 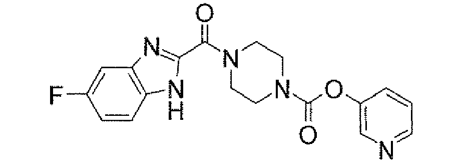 | ESI+: 370 |
| 11 | E1 | 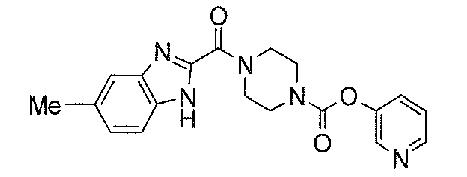 | FAB+: 366 |
| 12 | E1 | 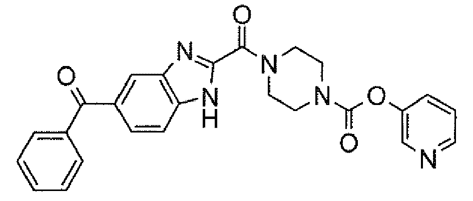 | FAB+: 456 |
| 13 | E1 | 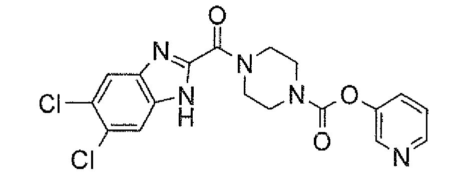 | FAB+: 420 |
| 14 | E1 | 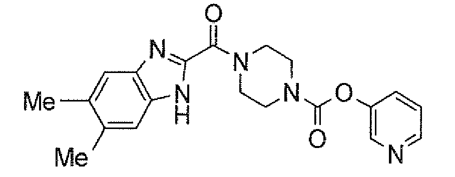 | ESI+: 380 |

**[Table 6]**

| | | | |
|---|---|---|---|
| 15 | E1 | 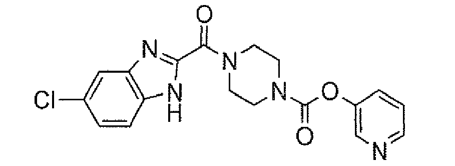 | ESI+: 386 |
| 16 | E1 | 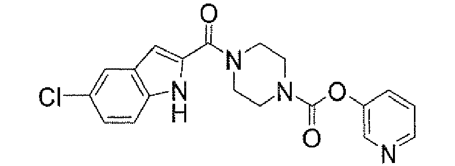 | ESI+: 385 |
| 17 | E1 | 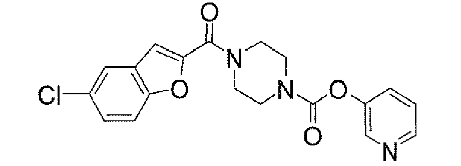 | ESI+: 386 |
| 18 | E1 | 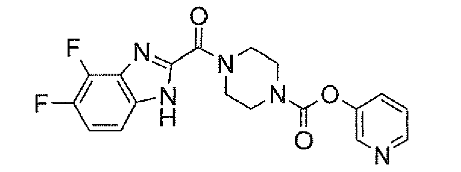 | ESI+: 388 |
| 19 | E1 | 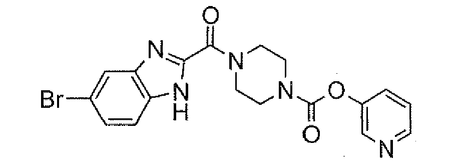 | ESI+: 430 |
| 20 | E1 | 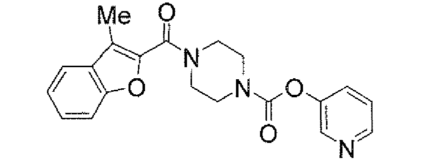 | ESI+: 366 |
| 21 | E1 | 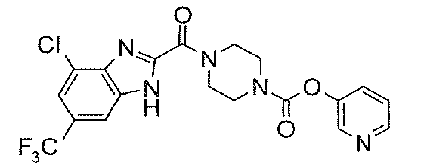 | ESI+: 454 |

**[Table 7]**

| | | | |
|---|---|---|---|
| 22 | E1 | 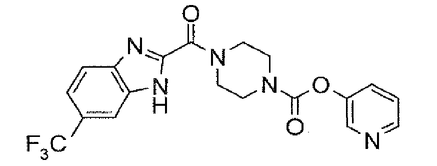 | ESI+: 420 |
| 23 | E1 | 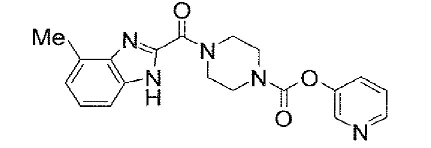 | FAB+: 366 |
| 24 | E2 | 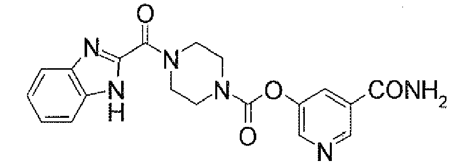 | FAB+: 395 |
| 25 | E2 | 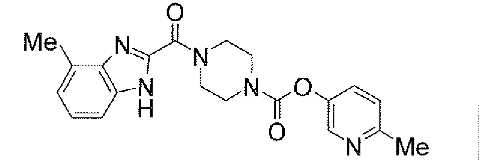 | ESI+: 380 |
| 26 | E2 | 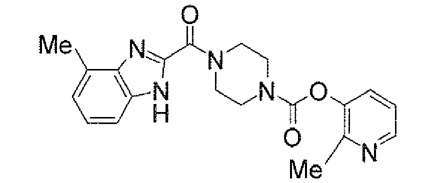 | ESI+: 380 |
| 27 | E2 | 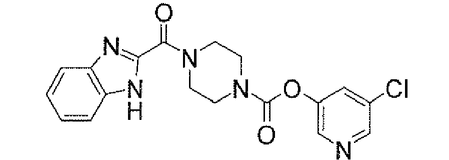 | ESI+: 386 |

**[Table 8]**

| Ex | Dat |
|---|---|
| 1 | NMR1: 3.61 (2H, brs), 3.78 (2H, brs), 3.84 (2H, brs), 4.61 (2H, brs), 7.27 (1H, t, J = 5.7 Hz), 7.34 (1H, t, J = 5.7 Hz), 7.45-7.49 (1H, m), 7.55 (1H, d, J = 3.0 Hz), 7.66-7.69 (1H, m), 7.76 (1H, d, J = 3.0 Hz), 8.44-8.47 (2H, m), 13.19 (1H, s); mp: 200-201°C (Hex/EtOAc) |
| 3 | NMR1: 3.51-3.90 (8H, m), 6.87 (1H, s), 7.06 (1H, m), 7.20 (1H, m), 7.43-7.48 (2H, m), 7.63 (1H, d, J = 8.2 Hz), 7.67 (1H, ddd, J = 1.4, 2.5, 8.2 Hz), 8.45-8.47 (2H, m), 11.62 (1H, s) |
| 4 | NMR1: 3.57-3.86 (8H, m), 7.44-7.49 (3H, m), 7.67 (1H, ddd, J = 1.4, 2.8, 8.3 Hz), 7.80 (1H, s), 7.95 (1H, m), 8.04 (1H, m), 8.44-8.47 (2H, m) |
| 5 | NMR1: 3.56 (2H, brs), 3.76 (6H, brs), 3.79 (3H, s), 6.72 (1H, s), 7.10 (1H, t, J = 5.8 Hz), 7.26 (1H, t, J = 5.8 Hz), 7.45-7.54 (2H, m), 7.61-7.70 (2H, m), 8.44-8.45 (2H, m) |
| 7 | NMR1: 3.56 (2H, brs), 3.75 (2H, brs), 3.85 (4H, brs), 7.35 (1H, t, J = 5.7 Hz), 7.45-7.49 (3H, m), 7.65-7.70 (2H, m), 7.77 (1H, d, J = 5.7 Hz), 8.44-8.46 (2H, m); mp: 126°C (Hex/EtOAc) |
| 8 | NMR1: 3.63 (2H, brs), 3.81 (4H, brs), 4.43 (2H, brs), 7.45-7.49 (1H, m), 7.57-7.69 (3H, m), 8.15-8.23 (2H, m), 8.44-8.47 (2H, m); mp: 145°C (Hex/EtOAc) |
| 9 | NMR1: 3.60 (2H, s), 3.80-3.84 (7H, m), 4.63 (2H, m), 6.8-7.0 (2H, m), 7.45-7.68 (3H, m), 8.44-8.46 (2H, m), 13.04 (1H, s) |
| 10 | NMR1: 3.61 (2H, brs), 3.78 (2H, brs), 3.83 (2H, brs), 4.57 (2H, brs), 7.14-7.20 (1H, m), 7.39-7.43 (1H, m), 7.45-7.49 (1H, m), 7.65-7.68 (2H, m), 8.44-8.46 (2H, m); mp: 230°C (Hex/EtOAc) |
| 13 | NMR1: 3.61-3.84 (6H, m), 4.52 (2H, s), 7.47 (1H, dd, J = 4.7,8.4 Hz), 7.66-7.69 (1H, m), 7.93 (2H, s), 8.45-8.47 (2H, m), 13.5 (1H, s); mp: 224-225°C (EtOAc) |
| 14 | NMR1: 2.33 (6H, s), 3.00-3.85 (6H, m), 4.64 (2H, s), 7.30-7.49 (3H, m), 7.66-7.69 (1H, m), 8.41-8.47 (2H, m); mp: 240-241°C (EtOAc) |
| 15 | NMR1: 3.55-3.90 (6H, m), 4.57 (2H, s), 7.24-7.90 (5H, m), 8.44-8.48 (2H, m), 13.35 (1H, s); mp: 225-226°C (EtOAc) |
| 17 | NMR1: 3.58 (2H, brs), 3.75 (2H, brs), 3.83 (4H, brs), 7.44 (1H, s), 7.45-7.51 (2H, m), 7.65-7.68 (1H, m), 7.74 (1H, d, J = 6.6 Hz), 7.85 (1H, d, J = 1.6 Hz), 8.44-8.46 (2H, m) |
| 18 | NMR1: 3.45-3.90 (6H, m), 4.52 (2H, s), 7.30-7.44 (2H, brs), 7.48 (1H, dd, J = 5.0,8.4 Hz), 7.64-7.70 (1H, m), 8.45-8.47 (2H, m), 13.62 (1H, s); mp: 245-247°C (EtOAc) |
| 19 | NMR1: 3.51-3.80 (6H, m), 4.57 (2H, s), 7.35-8.00 (5H, m), 8.44-8.48 (2H, m), 13.3-13.45 (1H, m) |
| 20 | NMR1: 2.32 (3H, s), 3.56 (2H, brs), 3.74 (6H, brs), 7.35 (1H, t, J = 5.4 Hz), 7.43-7.48 (2H, m), 7.61 (1H, d, J = 6.3 Hz), 7.64-7.67 (1H, m), 7.73 (1H, d, J = 5.8 Hz), 8.44-8.46 (2H, m); mp: 124°C (Hex/EtOAc) |

**[Table 9]**

| | |
|---|---|
| 21 | NMR1: 3.50-4.60 (8H, m), 7.48 (1H, dd, J = 4.7,8.4 Hz), 7.64-7.70 (1H, m), 7.72-8.20 (2H, m), 8.42-8.50 (2H, m), 14.0 (1H, br) |
| 22 | NMR1: 3.51-3.88 (6H, m), 4.56 (2H, m), 7.49 (1H, dd, J = 4.7,8.3 Hz), 7.51-8.25 (4H, m), 8.43-8.50 (2H, m), 13.63 (1H, s) |
| 23 | NMR1: 2.57 (3H, s), 3.61 (2H, brs), 3.78 (2H, brs), 3.85 (2H, brs), 4.50 (1H, brs), 4.64 (1H, brs), 7.05-7.13 (1H, m), 7.12-7.24 (1H, m), 7.34-7.57 (2H, m), 7.66-7.69 (1H, m), 8.44-8.47 (2H, m), 13.11 (0.5H, s), 13.22 (0.5H, s); mp: 213°C (acetone) |

**[Table 10]**

| Cpd | Str | Cpd | Str |
|---|---|---|---|
| 1 | 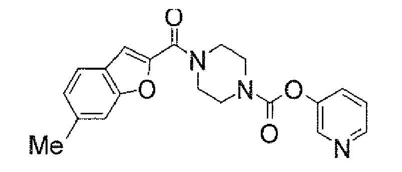 | 49 | 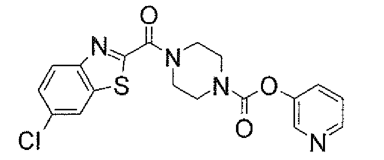 |
| 2 | 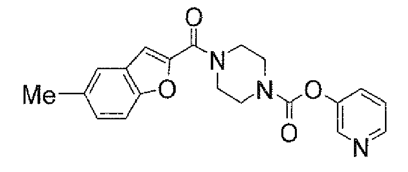 | 50 | 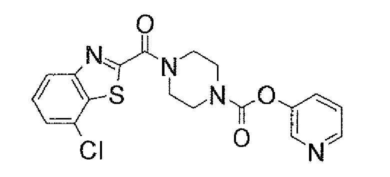 |
| 3 | 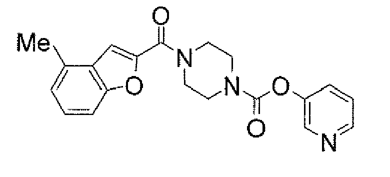 | 51 | 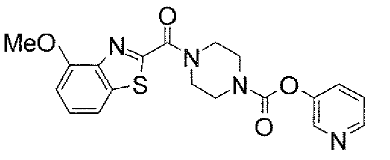 |
| 4 | 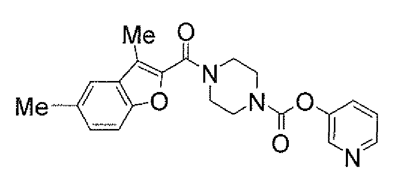 | 52 | 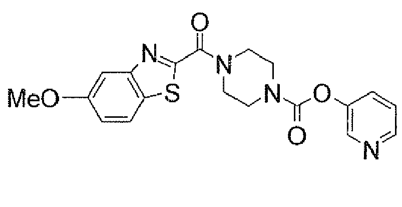 |
| 5 | 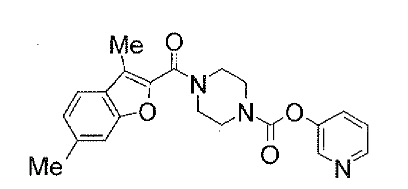 | 53 | 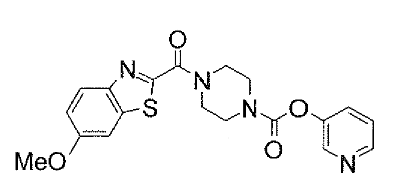 |
| 6 | 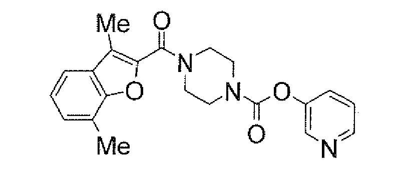 | 54 | 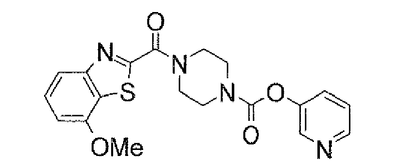 |
| 7 | 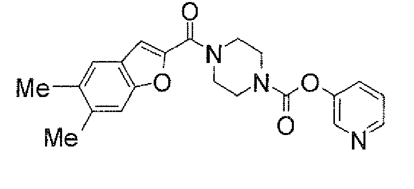 | 55 | 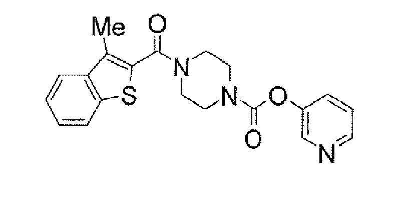 |

**[Table 11]**

| | | | |
|---|---|---|---|
| 8 | 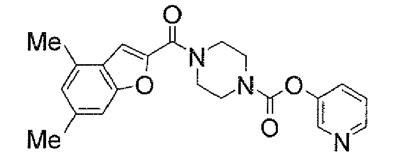 | 56 | 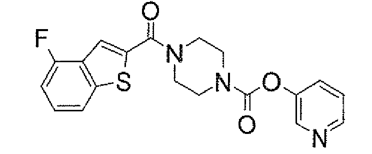 |
| 9 | 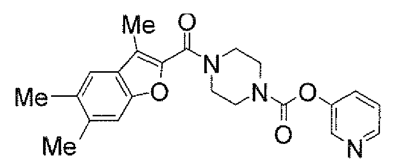 | 57 | 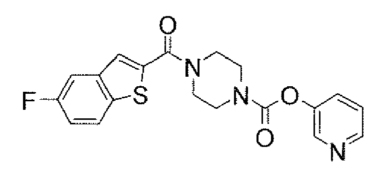 |
| 10 | 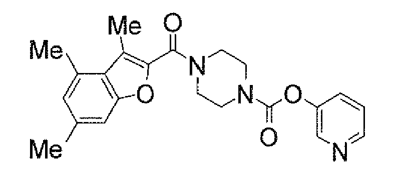 | 58 | 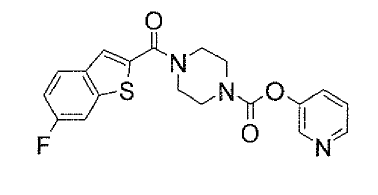 |
| 11 | 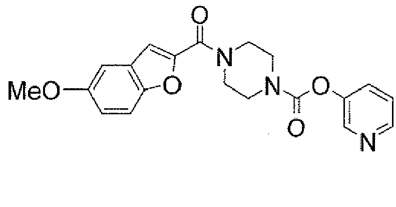 | 59 | 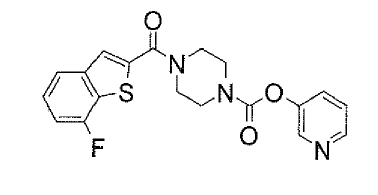 |
| 12 | 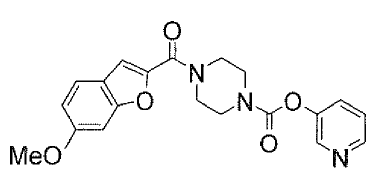 | 60 | 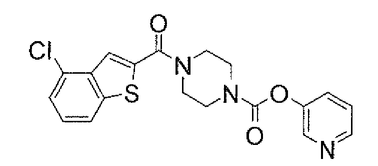 |
| 13 | 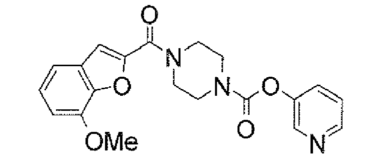 | 61 | 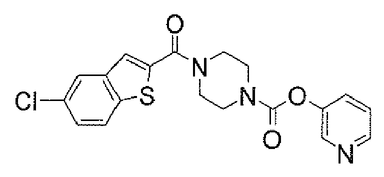 |
| 14 | 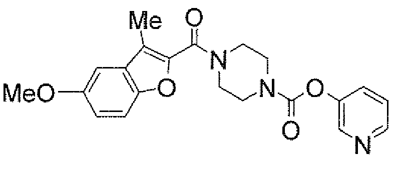 | 62 | 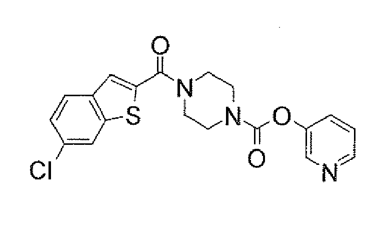 |

**[Table 12]**

| | | | |
|---|---|---|---|
| 15 | 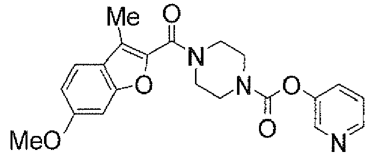 | 63 | 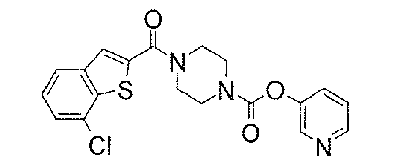 |
| 16 | 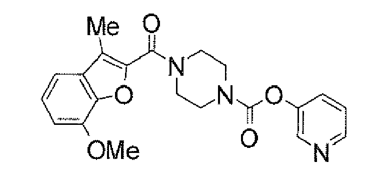 | 64 | 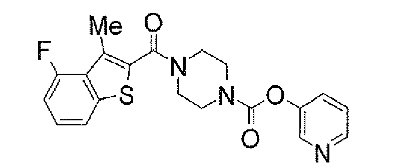 |
| 17 | 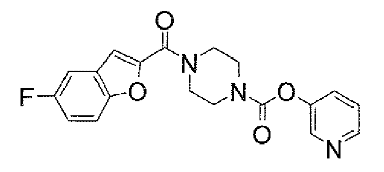 | 65 | 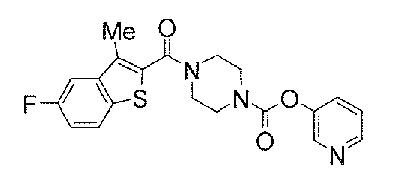 |
| 18 | 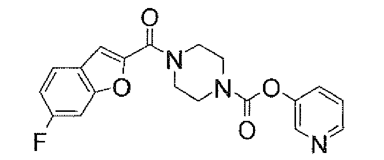 | 66 | 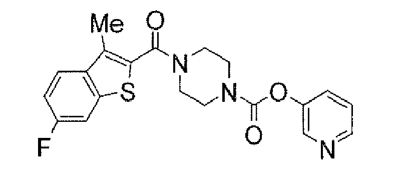 |
| 19 | 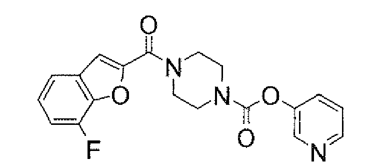 | 67 | 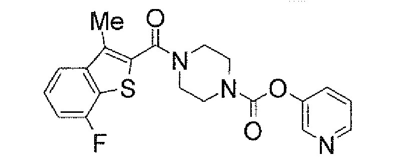 |
| 20 | 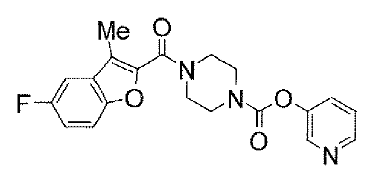 | 68 | 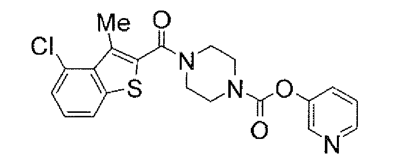 |
| 21 | 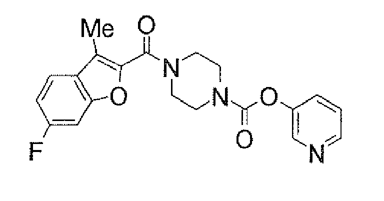 | 69 | 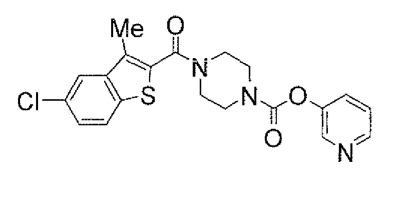 |

**[Table 13]**

| | | | |
|---|---|---|---|
| 22 | 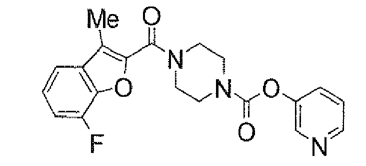 | 70 | 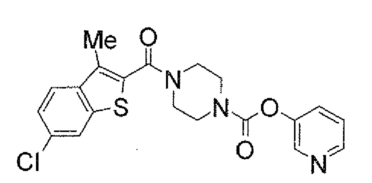 |
| 23 | 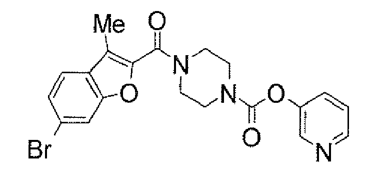 | 71 | 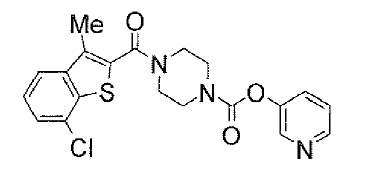 |
| 24 | 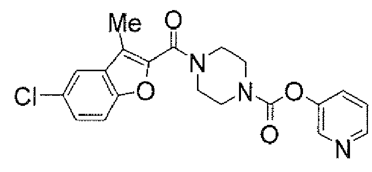 | 72 | 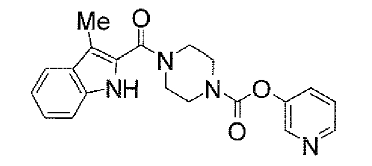 |
| 25 | 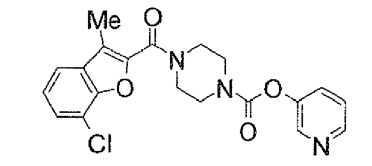 | 73 | 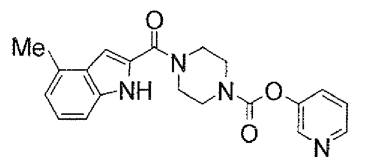 |
| 26 | 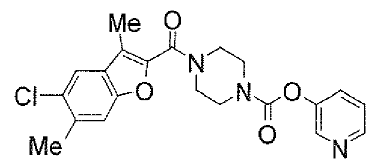 | 74 | 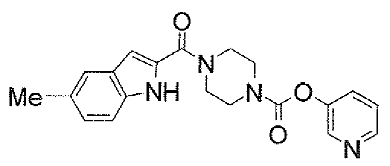 |
| 27 | 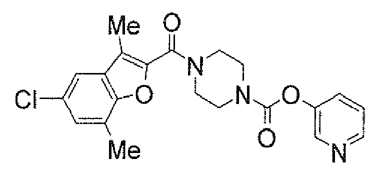 | 75 | 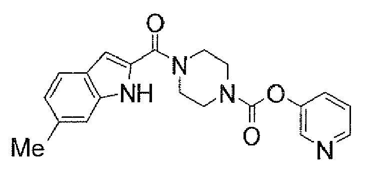 |
| 28 | 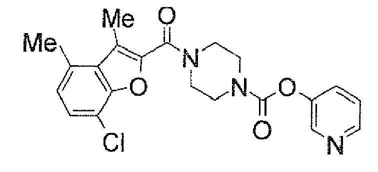 | 76 | 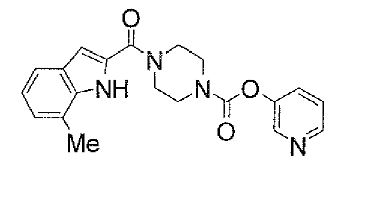 |

**table 14]**

| | | | |
|---|---|---|---|
| 29 | 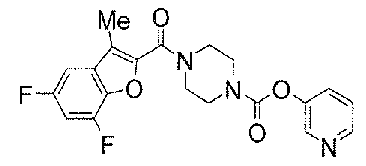 | 77 | 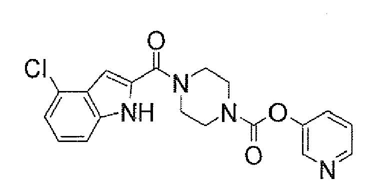 |
| 30 | 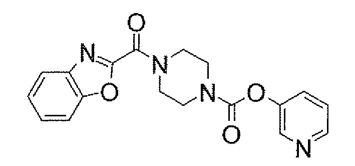 | 78 | 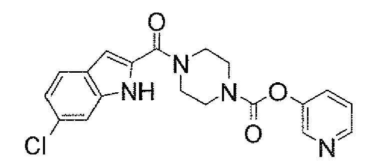 |
| 31 | 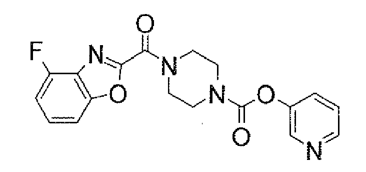 | 79 | 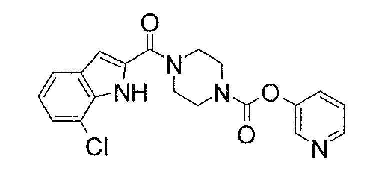 |
| 32 | 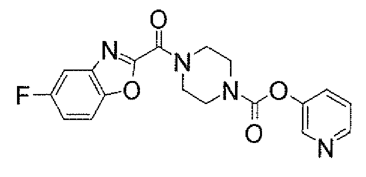 | 80 | 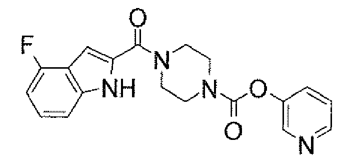 |
| 33 | 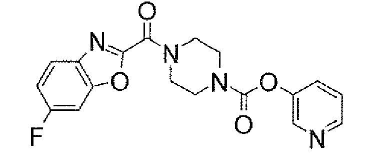 | 81 | 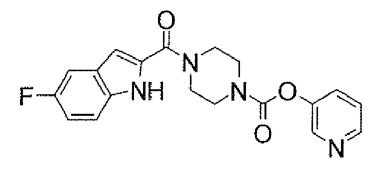 |
| 34 | 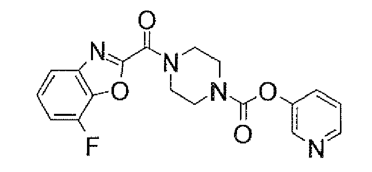 | 82 | 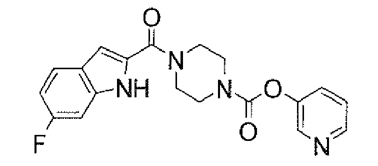 |
| 35 | 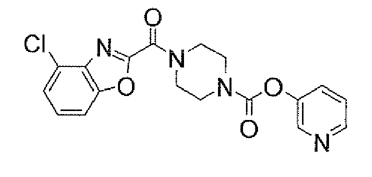 | 83 | 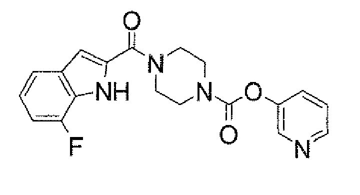 |

**[Table 15]**

| | | | |
|---|---|---|---|
| 36 | 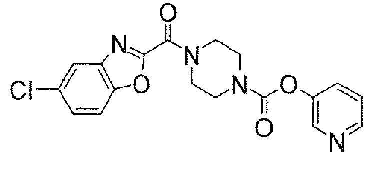 | 84 | 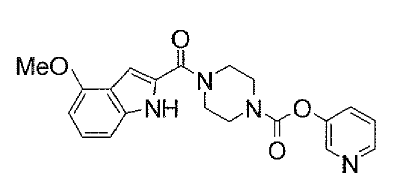 |
| 37 | 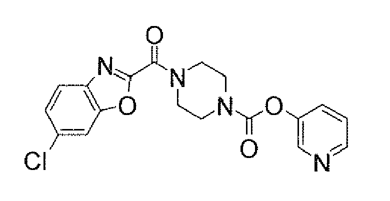 | 85 | 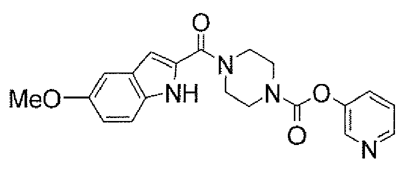 |
| 38 | 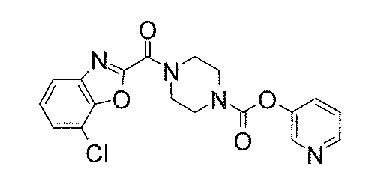 | 86 | 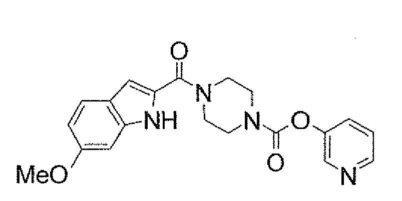 |
| 39 | 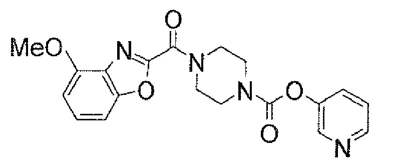 | 87 | 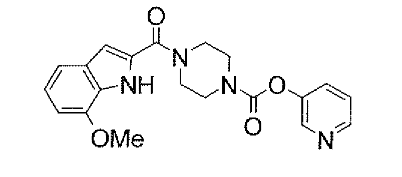 |
| 40 | 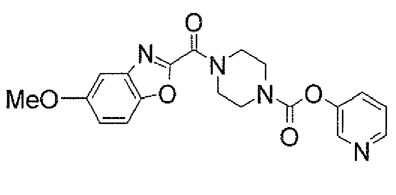 | 88 | 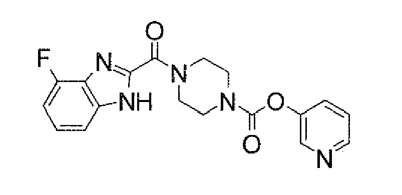 |
| 41 | 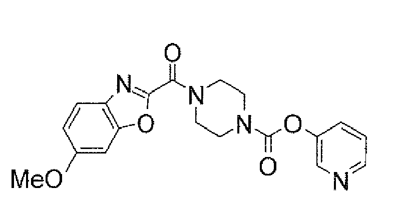 | 89 | 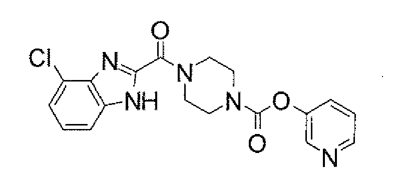 |
| 42 | 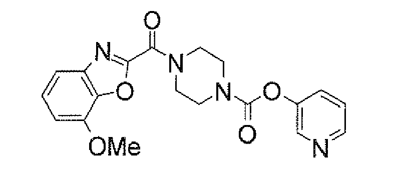 | 90 | 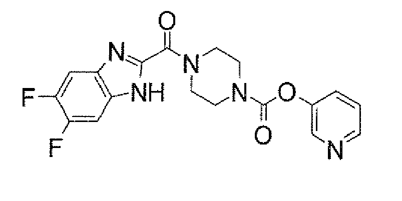 |

**Table 16]**

| | | | |
|---|---|---|---|
| 43 | 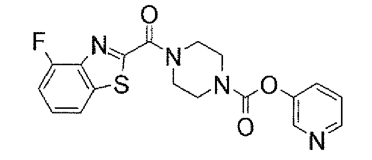 | 91 | 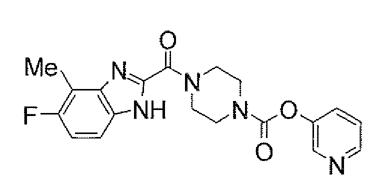 |
| 44 | 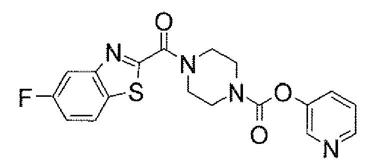 | 92 | 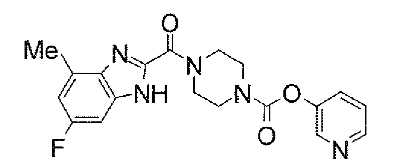 |
| 45 | 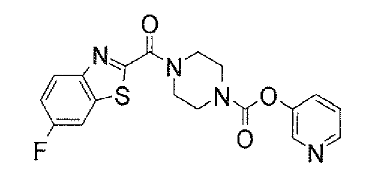 | 93 | 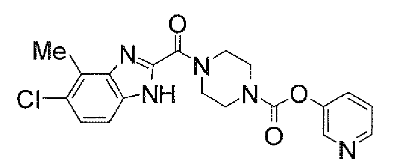 |
| 46 | 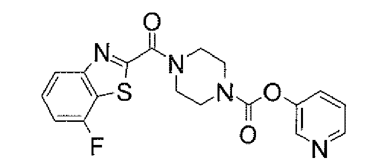 | 94 | 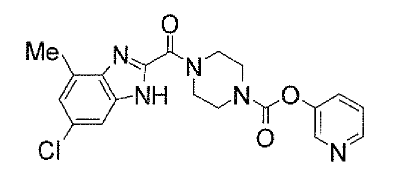 |
| 47 | 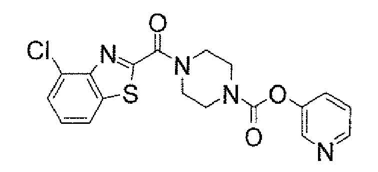 | 95 | 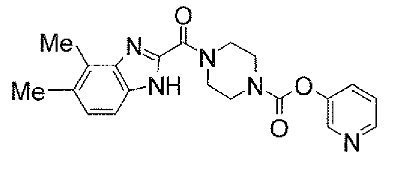 |
| 48 | 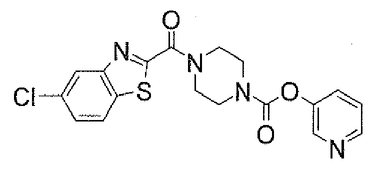 | 96 | 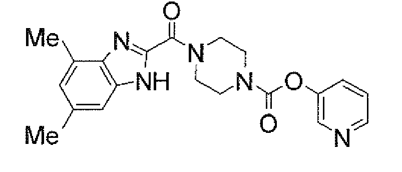 |

### Industrial Applicability

The compound of the formula (I) or a salt thereof has an excellent FAAH receptor inhibitory action, and has an action to increase the effective bladder capacity, an action to ameliorate sleep disorders, an anti-diuretic action, and an analgesic activity on lower urinary tract pain including bladder pain and the like, based on the inhibitory action. Thus, it can be used as an agent for preventing and/or treating FAAH-related diseases, particularly nocturia, interstitial cystitis, painful bladder syndrome, or chronic non-bacterial prostatitis/chronic pelvic pain syndrome.

## Claims

1. A compound of the formula (I) or a salt thereof: 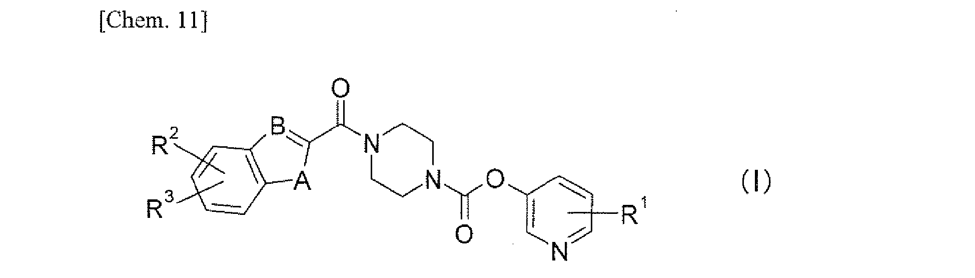 (wherein
A is NR⁴, S, or O,
B is CR⁵ or N,
R¹ is R⁰, halogen, -CO-O-lower alkyl, or -CO-N(R⁰)₂,
R² and R³ are the same as or different from each other and represent R⁰, -OR⁰, -O-benzyl, halogen, halogeno-lower alkyl, or -CO-aryl,
R⁴ and R⁵ are the same as or different from each other and represent R⁰, and
R⁰ are the same as or different from each other and represent H or lower alkyl).

2. The compound or a salt thereof according to claim 1, wherein the compound is selected from the group consisting of:
pyridin-3-yl 4-(1,3-benzothiazol-2-ylcarbonyl)piperazine-1-carboxylate,
pyridin-3-yl 4-[(5,6-dichtoro-1H-benzimidazol-2-yl)carbonyl]piperazine-1-carboxylate,
pyridin-3-yl 4-[(4,5-difluoro-1H-benzimidazol-2-yl)carbonyl]piperazine-1-carboxylate,
pyridin-3-yl 4-[(3-methyl-1-benzofuran-2-yl)carbonyl]piperazine-1-carboxylate,
pyridin-3-yl 4-[(4-methyl-1-H-benzimidazol-2-yl)carbonyl]piperazine-1-carboxylate,
pyridin-3-yl 4-(1H-benzimidazol-2-ylcarbonyl)piperazine-1-carboxylate,
pyridin-3-yl 4-[(1-methyl-1H-indol-2-yl)carbonyl]piperazine-1-carboxylate,
pyridin-3-yl 4-(1-benzofuran-2-ylcarbonyl)piperazine-1-carboxylate,
pyridin-3-yl 4-[(5-fluoro-1H-benzimidazol-2-yl)carbonyl]piperazine-1-carboxylate,
pyridin-3-yl 4-[(5-methyl-1H-benzimidazol-2-yl)carbonyl]piperazine-1-carboxylate,
pyridin-3-yl 4-[(5,6-dimethyl-1H-benzimidazol-2-yl)carbonyl]piperazine-1-carboxylate, and
pyridin-3-yl 4-[(5-chloro-1H-benzimidazol-2-yl)carbonyl]piperazine-1-carboxylate.

3. A pharmaceutical composition comprising the compound or a salt thereof according to claim 1 and a pharmaceutically acceptable excipient.

4. A pharmaceutical composition for treating nocturia, interstitial cystitis, painful bladder syndrome, or chronic non-bacterial prostatitis/chronic pelvic pain syndrome, comprising the compound or a salt thereof according to claim 1.

5. Use of the compound or a salt thereof according to claim 1 for the manufacture of a pharmaceutical composition for treating nocturia, interstitial cystitis, painful bladder syndrome, or chronic non-bacterial prostatitis/chronic pelvic pain syndrome.

6. Use of the compound or a salt thereof according to claim 1 for treating nocturia, interstitial cystitis, painful bladder syndrome, or chronic non-bacterial prostatitis/chronic pelvic pain syndrome.

7. A method for treating nocturia, interstitial cystitis, painful bladder syndrome, or chronic non-bacterial prostatitis/chronic pelvic pain syndrome, comprising administering to a patient an effective amount of the compound or a salt thereof according to claim 1.
